# Europäisches Patentamt
## European Patent Office
### Office européen des brevets

(19)

(11) Veröffentlichungsnummer: **0 209 731**
A2

(12) # EUROPÄISCHE PATENTANMELDUNG

(21) Anmeldenummer: 86108386.3

(22) Anmeldetag: 19.06.86

(51) Int. Cl.⁴: **C07C 55/14** , C07C 55/20 , C07C 63/24 , C07C 63/28 , C07C 51/41 , C07C 93/02 , //C08G69/40

(30) Priorität: 21.06.85 DE 3522215

(43) Veröffentlichungstag der Anmeldung:
26.01.87 Patentblatt 87/05

(84) Benannte Vertragsstaaten:
BE CH DE FR GB IT LI NL

(71) Anmelder: BASF Aktiengesellschaft
Carl-Bosch-Strasse 38
D-6700 Ludwigshafen(DE)

(72) Erfinder: Pilz, Georg
Ahornweg 4
D-6730 Neustadt(DE)
Erfinder: Bürger, Ger, Dr.
Hoeferstrasse 16
D-6800 Mannheim 23(DE)
Erfinder: Barl, Manfred, Dr.
Pappelstrasse 2
D-6701 Otterstadt(DE)
Erfinder: Thiel, Gerhard
Untergasse 35
D-6700 Ludwigshafen(DE)

(54) Salze aus Oxaalkylendiaminen mit Dicarbonsäuren, deren Herstellung und Verwendung sowie daraus hergestellte Polyamide.

(57) Salze aus Oxaalkylendiamien der Formel I

$H_2N-(R^1-O)_n-R^2-NH_2$ I

in der $R^1$ und $R^2$ gleich oder verschieden sein können und jeweils für einen Alkylenrest mit 2 bis 4 Kohlenstoffatomen stehen und n eine ganze Zahl von 1 bis 3 bedeutet, und Alkandicarbonsäuren mit 4 bis 12 Kohlenstoffatomen Terephthalsäure oder Isophthalsäure, gekennzeichnet durch einen Gehalt an Hydrazin.

EP 0 209 731 A2

## Salze aus Oxaalkylendiaminen mit Dicarbonsäuren, deren Herstellung und Verwendung sowie daraus hergestellte Polyamide

Da an Polyamide, hergestellt aus Dicarbonsäuren und Diaminen, hohe Ansprüche hinsichtlich ihrer Qualität gestellt werden, trifft dies auch auf die für die Herstellung solcher Polyamide verwendeten Salze aus Dicarbonsäuren und Diaminen zu. Aus der US-PS 4 165 335 ist bereits bekannt, daß man bei der Herstellung von Salzen aus Alkandicarbonsäuren und Alkandiaminen den Alkandiaminen Hydrazinhydrat zusetzt um die Eigenfarbe zu verbessern. Es ist auch bereits bekannt, Polyamide, aufgebaut aus Alkandicarbonsäuren und Oxoalkylendiaminen wie 4,7-Dioxadecan-1,10-diamin aus den entsprechenden Salzen herzustellen. Obwohl man bereits sehr große Sorgfalt auf die Reinigung solcher Diamine, z.B. durch Destillation, legt, sind die aus Oxaalkylendiaminen hergestellten Salze hinsichtlich der erforderlichen Kennzahlen verbesserungsbedürftig. Dies gilt insbesondere für die Periodatzahl und die UV-Zahl der erzeugten Salze nach einer Wärmebehandlung. Die nachteiligen Eigenschaften werden durch geringste Mengen an Verunreinigungen hervorgerufen, deren Natur bislang nicht bekannt ist und von den jeweiligen Herstellungsverfahren abhängen und die mit den herkömmlichen Reinigungsmethoden unter vertretbarem Aufwand bislang nicht entfernt werden konnten.

Es war deshalb die technische Aufgabe gestellt, Salze aus Dicarbonsäuren und Oxaalkylendiaminen zur Verfügung zu stellen, die hinsichtlich ihrer Kennzahlen, insbesondere der Periodatzahl und UV-Zahl der Salze nach einer Wärmebehandlung sowie weiteren Kennzahlen den gestellten Anforderungen besser entsprechen und es ermöglichen, daraus Polyamide mit verbesserter Qualität zu erzeugen.

Diese Aufgabe wird gelöst durch Salze aus Oxaalkylendiaminen der Formel I

$H_2N-(R^1-O)_n-R^2-NH_2$ in der $R^1$ und $R^2$ gleich oder verschieden sein können und jeweils einen Alkylenrest mit 2 bis 4 Kohlenstoffatomen bezeichnen und n eine ganze Zahl von 1 bis 3 bedeutet und Alkandicarbonsäuren mit 4 bis 12 Kohlenstoffatomen Terephthalsäure oder Isophthalsäure, gekennzeichnet durch einen Gehalt an Hydrazin.

Es wurde ferner gefunden, daß man Salze aus Oxaalkylendiaminen der Formel I und Alkandicarbonsäuren mit 4 bis 12 Kohlenstoffatomen, Terephthalsäure oder Isophthalsäure durch Umsetzen der genannten Ausgangsverbindungen in Lösung vorteilhaft erhält, wenn man den als Ausgangsverbindung verwendeten Dicarbonsäuren und/oder Oxaalkylendiaminen Hydrazin in Form von Hydrazinhydrat zusetzt.

Weiter ist ein Gegenstand der Erfindung die Verwendung der vorgenannten Salze zur Herstellung von Polyamiden und die daraus hergestellten Polyamide selbst.

Die erfindungsgemäßen Salze aus Dicarbonsäuren und Oxaalkylendiaminen mit einem Gehalt an Hydrazin haben den Vorteil, daß auf einfache Weise nicht nur die Farbzahl und Vergilbungszahl verbessert wird sondern die Periodatzahl in unerwartetem Maß gesenkt wird und darüberhinaus auch die UV-Zahl nach einer Wärmebehandlung der Salze, wie sie in der Technik erforderlich ist, sowie weitere Kennzahlen verbessert werden.

Als Ausgangsstoffe verwendet man Oxaalkylendiamine der Formel I

$H_2N-(R^1-O)_n-R^2-NH_2$ I

in der $R^1$ und $R^2$ gleich oder verschieden sein können und jeweils einen Alkylenrest mit 2 bis 4 Kohlenstoffatomen bezeichnen. Geeignete Alkylenreste sind beispielsweise Ethylenreste, Propylen-1,2-Reste, Propylen-1,3-Reste oder Butylen-1,4-Reste. Besonders bevorzugt sind Alkylenreste mit 2 bis 3 Kohlenstoffatomen. In der Formel I bezeichnet n eine ganze Zahl von 1 bis 3, insbesondere 1 oder 2. Geeignete Oxaalkylendiamine sind beispielsweise solche der Formeln II bis VII

$H_2N-(CH_2)_2-O-(CH_2)_3-O-(CH_2)_2-NH_2$ II

$H_2N-CH_2-CH_2-O-CH_2-CH_2-NH_2$ III

$H_2N-[CH_2-CH_2-O]_2-CH_2-CH_2-NH_2$ IV

$H_2N-(CH_2)_4-O-(CH_2)_4-NH_2$ V

$H_2N-(CH_2)_3-O-(CH_2)_4-O-(CH_2)_3-NH_2$

$H_2N-(CH_2)_3-O-(CH_2)_2-O-(CH_2)_3-NH_2$ VII

Besondere technische Bedeutung hat ein Oxaalkylendiamin der Formel VII erlangt.

Als Ausgangsstoffe verwendet man ferner Alkandicarbonsäuren mit 4 bis 12 Kohlenstoffatomen. Bevorzugt sind $\alpha,\omega$-Alkandicarbonsäuren, insbesondere solche mit gerader Kohlenstoffkette. Geeignete Dicarbonsäuren sind beispielsweise Glutarsäure, Adipinsäure, Korksäure, Sebacinsäure oder Dodecandisäure. Besondere technische Bedeutung haben Adipinsäure und Sebacinsäure erlangt. Ferner sind geeignete Dicarbonsäuren Terephthalsäure und Isophthalsäure sowie deren Gemische.

Es versteht sich, daß aus dem bevorzugten Ausgangsstoff die bevorzugten Endprodukte resultieren.

Salze aus den genannten Oxaalkylendiaminen und . Dicarbonsäuren werden in der Regel in Lösung, z.B. Wasser, Methanol oder Ethanol, insbesondere wäßriger Lösung, hergestellt. Hierbei geht man beispielsweise von wäß-rigen Lösungen von Dicarbonsäuren, die einen Gehalt von mehr als 40 Gew.%, insbesondere mehr als 50 Gew.%, haben aus. Solche Lösungen werden dann mit einer wäßrigen Lösung, z.B. 50 bis 60 gew.%ige wäßrige Lösung von Oxaalkylendiaminen oder geschmolzenen Oxaalkylendiaminen, umgesetzt. Es ist auch möglich, von einer wäßrigen Lösung von Diaminen, die vorteilhaft einen Gehalt von über 50 Gew.% haben, auszugehen und diese wäßrige Lösung mit festen Alkandicarbonsäuren umzusetzen. Bei der Umsetzung hält man in der Regel Temperaturen von 20 bis 100°C, insbesondere von 60 bis 90°C, ein. Im Hinblick auf die Verwendung für die Polykondensation versteht es sich, daß man Dicarbonsäuren und Diamine in stöchiometrischen Mengen anwendet.

Man erhält so wäßrige Lösungen der entsprechenden Salze aus Dicarbon-säuren und Oxaalkylendiaminen mit einem Gehalt von beispielsweise 50 bis 60 Gew.%. Aus der wäßrigen Lösung erhält man z.B. durch Abkühlen und Ein-dampfen die entsprechenden Salze selbst. Es ist aber auch möglich, die wäßrigen Lösungen, wie sie bei der Herstellung anfallen, direkt für die Herstellung von Polyamiden zu verwenden. Demzufolge sind unter Salzen aus Dicarbonsäuren und Oxaalkylendiaminen auch für die Herstellung von Polyamiden geeignete wäßrige Lösungen, die solche Salze enthalten, zu verstehen.

Ein wesentliches Merkmal der Erfindung ist es, daß den Ausgangsstoffen, d.h. den Dicarbonsäuren und/oder Oxaalkylendiaminen Hydrazin in Form von Hydrazinhydrat zugesetzt wird. Vorteilhaft setzt man Hydrazinhydrat den flüssigen Ausgangsstoffen, z.B. den wäßrigen Lösungen von Dicarbonsäuren oder Oxaalkylendiaminen oder den geschmolzenen Oxaalkylendiaminen zu.

Besonders vorteilhaft hat es sich erwiesen, Hydrazinhydrat den geschmolzenen oder in wäßriger Lösung vorliegenden Oxaalkylendiaminen zuzusetzen. Vorzugsweise hält man einen Gehalt an Hydrazin von 5 bis 200 ppm, bezogen auf das Diamin, in Form von Hydrazinhydrat ein. Besonders bewährt hat sich ein Zusatz von 10 bis 150 ppm Hydrazin in Form von Hydrazinhydrat. Es versteht sich, daß die jeweiligen Salze aus Alkandicarbonsäuren und Oxaalkylendiaminen das bei der Herstellung zugesetzte Hydrazin in Form von Hydrazinhydrat enthalten. In welcher Form Hydrazinhydrat in den erhaltenen Salzen vorliegt, ist nicht bekannt.

Aus den so erhaltenen Salzen oder wäßrigen Lösungen erhält man durch Polykondensation die entsprechenden Polyamide. Die Polykondensation wird beispielsweise durchgeführt, indem man die Salze oder die wäßrige Lösung auf Temperaturen von 150 bis 220°C erhitzt, das entstehende Wasser abführt und anschließend die erhaltene Schmelze gegebenenfalls unter vermindertem Druck bei polyamidbildenden Temperaturen, insbesondere bei einer Temperatur von 220 bis 275°C polykondensiert.

Polyamide eignen sich zur Herstellung von geformten Massen wie Fasern, Formteilen, Folien und Überzügen.

Die Erfindung sei an folgenden Beispielen veranschaulicht.

Beispiel

Adipinsäure in Form einer 50 gew.%igen Lösung wird bei 90°C mit einer äquivalenten Menge an 4,7-Dioxadecan-1,10-diamin die Hydrazin in Form von Hydrazinhydrat enthält, umgesetzt. Die mit unterschiedlichen Mengen an Hydrazin erzielten Ergebnisse werden in folgender Tabelle aufgeführt.

Tabelle

|  | ohne Zusatz | 25 ppm Hydrazin | 50 ppm Hydrazin | 100 ppm Hydrazin |
|---|---|---|---|---|
| Eigenfarbe (1) | 7 | 5 | 4 | 2 |
| Vergilbung (2) | 156 | 60 | 23 | 16 |
| UV-Zahl (3) | 446 | 374 | 273 | 227 |
| Perjodatzahl (4) | 0,111 | 0,055 | 0,021 | 0,007 |
| UV-Zahl nach 30 min bei 90° C | 447 | 469 | 365 | 275 |

1) Eigenfarbe: APHA gemessen bei 90°C an der 40 gew.%igen wäßrigen Lösung

2) Vergilbung: APHA einer 40 gew.%igen wäßrigen Lösung nach 24stündiger Temperierung auf 85°C. Bestimmung der Extinktion mittels eines Elko II Photometers mit S 47 und J 62 Filter bei einer Schichtdicke von 5 cm. Mittels einer Eichkurve ergibt sich APHA aus Extinktion S 47 minus Extinktion J 62.

3) UV-Zahl: Summe der Extinktionen bei 226, 282 und 295 m$\mu$ x 100, gemessen an einer 40 gew.%igen wäßrigen Lösung bei 25°C gegen bidestilliertes Wasser in 10 cm Schichtdicke.

4) Perjodatzahl: 50 g einer 40 gew.%igen wäßrigen Lösung des Salzes werden mit 1 ml einer 0,5 gew.%igen wäßrigen Kaliumperjodatlösung versetzt, 30 Minuten auf 90°C erhitzt, dann auf Raumtemperatur abgekühlt und in einer Schichtdicke von 5 cm mit einem Elko II Photometer mit einem Filter S 45 die Extinktion gemessen

**Ansprüche**

1. Salze aus Oxaalkylendiamien der Formel I

H$_2$N-(R$^1$-O)$_n$-R$^2$-NH$_2$ I

in der R$^1$ und R$^2$ gleich oder verschieden sein können und jeweils für einen Alkylenrest mit 2 bis 4 Kohlenstoffatomen stehen und n eine ganze Zahl von 1 bis 3 bedeutet, und Alkandicarbonsäuren mit 4 bis 12 Kohlenstoffatomen, Terephthalsäure oder Isophthalsäure, gekennzeichnet durch einen Gehalt an Hydrazin.

2. Salze nach Anspruch 1, gekennzeichnet durch einen Gehalt von 5 bis 200 ppm Hydrazin, bezogen auf Oxaalkylendiamin.

3. Salze aus Oxaalkylendiaminen der Formel VII

H$_2$N-(CH$_2$)$_3$-O-(CH$_2$)$_2$-O-(CH$_2$)$_3$-NH$_2$ VII

und Adipinsäure oder Sebacinsäure, gekennzeichnet durch einen Gehalt von 5 bis 200 ppm Hydrazin, bezogen auf das Diamin.

4. Verfahren zur Herstellung von Salzen aus Oxaalkylendiaminen der Formel I und Dicarbonsäuren durch Umsetzung von Oxaalkylendiaminen der Formel I mit Dicarbonsäuren in Lösung, dadurch gekennzeichnet, daß man den als Ausgangsstoffen verwendeten Dicarbonsäuren und/oder Oxaalkylendiaminen Hydrazin in Form von Hydrazinhydrat zusetzt.

5. Verfahren nach Anspruch 4, dadurch gekennzeichnet, daß man 5 bis 200 ppm Hydrazin in Form von Hydrazinhydrat, bezogen auf das Oxaalkylendiamin zusetzt.

6. Verfahren nach den Ansprüchen 4 und 5, dadurch gekennzeichnet, daß man Hydrazin in Form von Hydrazinhydrat den Oxaalkylendiaminen zusetzt.

7. Verwendung von Salzen nach den Ansprüchen 1 bis 6 zur Herstellung von Polyamiden.

8. Polyamide, hergestellt durch Kondensation von Salzen aus Oxamethylendiaminen der Formel I

H$_2$N-(R$^1$-O)$_n$-R$^2$-NH$_2$ I

in der R$^1$ und R$^2$ gleich und/oder verschieden sein können und jeweils einen Alkylenrest mit 2 bis 4 Kohlenstoffatomen bezeichnen und n eine ganze Zahl von 1 bis 3 bedeutet und Alkandicarbonsäuren mit 4 bis 12 Kohlenstoffatomen, Terephthalsäure oder Isophthalsäure mit einem Gehalt von 5 bis 200 ppm Hydrazin, bezogen auf das Oxaalkylendiamin, bei Polyamid-bildenden Temperaturen.